# EUROPÄISCHE PATENTSCHRIFT

(11) **EP 2 867 625 B1**
(45) Veröffentlichungstag und Bekanntmachung des Hinweises auf die Patenterteilung: **04.01.2017**
(21) Anmeldenummer: 13732360.6
(22) Anmeldetag: 21.06.2013
(51) Int. Cl.: G01D 5/34

(54) **VORRICHTUNG UND VERFAHREN ZUR BESTIMMUNG VON RELATIVVERLAGERUNGEN VON KÖRPERTEILEN ODER KÖRPERBEREICHEN**
DEVICE FOR AND METHOD OF DETERMINING RELATIVE DISPLACEMENT OF BODY PARTS OR AREAS
DISPOSITIF ET PRODÉDÉ POUR LA DÉTERMINATION D'UN DÉPLACEMENT RELATIF D'UNE PARTIE D'UN CORPS

(30) Priorität: 27.06.2012 DE 102012012695
(43) Veröffentlichungstag der Anmeldung: 06.05.2015
(73) Patentinhaber: Otto Bock HealthCare GmbH, 37115 Duderstadt (DE)
(72) Erfinder: KRUKENBERG, Jörg, 37115 Duderstadt (DE); VON MARCARD, Timo, 30627 Hannover (DE)
(74) Vertreter: Stornebel, Kai
(86) Internationale Anmeldenummer: PCT/EP2013/001845
(87) Internationale Veröffentlichungsnummer: WO 2014/000876

(56) Entgegenhaltungen:
- DE-A1-102009 005 536
- FR-A1- 2 954 493
- GB-A- 1 498 409
- US-A- 4 555 625
- US-A- 5 376 785

## Beschreibung

Die Erfindung betrifft eine Vorrichtung und ein Verfahren zur Bestimmung von Relativverlagerungen von Körperteilen oder Körperbereichen mit einem Sender und einem dem Sender zugeordneten Sensor. Die Erfindung ist insbesondere dazu geeignet, die Wirbelsäulentorsion oder Wirbelsäulenrotation während der Bewegung zu erfassen.

Zur Erfassung von Bewegungsdaten und Bewegungsabläufen ist neben einer reinen Bildaufnahme mittels einer oder mehrerer Kameras ein Verfahren bekannt, bei dem Marker auf die Körperoberfläche eines Menschen aufgebracht werden. Der sich bewegende Mensch wird mit einer Kamera aufgenommen, wobei die Marker an den Gelenkpunkten angeordnet sind. Die Bewegungen der Gelenkpunkte zueinander werden nach der Erfassung durch die Kameraeinrichtungen als Modell in einem Computer umgewandelt und auf einem Bildschirm abgebildet. Aus dem Model lassen sich Relativbewegungen von Körperteilen oder Körperbereichen zueinander errechnen, wobei der Berechnung Modellannahmen zugrunde liegen. So wird die Wirbelsäule zumeist als Stab angenommen, an dem das Becken und die Schultern festgelegt sind.

Das letztgenannte Verfahren benötigt einen hohen apparativen Aufwand, ist in der Regel nur in einer speziellen Umgebungssituation anwendbar und lässt keine oder nur ungenaue Rückschlüsse über die tatsächliche Verdrehung innerhalb der Wirbelsäule oder der tatsächlichen Verlagerung einzelner Körperbereiche oder Körperteile zueinander zu.

Die DE 10 2009 005 536 A1 betrifft einen Drehwinkelsensor zur berührungslosen Bestimmung des Drehwinkels einer Drehachse mit einer optischen Strahlungsquelle und einem Detektor, wobei im Strahlengang zwischen der Strahlungsquelle und dem Detektor eine Abschattungsstruktur auf einer Scheibe angeordnet ist, deren Position sich mit der Drehachse verändert. Die Abschattungsstruktur wird zumindest teilweise von der Strahlung der Strahlungsquelle in Abhängigkeit von dem Drehwinkel der Drehachse beleuchtet und auf den Detektor abgebildet. Auf diese Weise können Messfehler vermieden bzw. verringert und der zu erfassende Drehwinkel der Drehachse exakter ermittelt werden.

Die FR 2 954 493 A1 betrifft einen optischen Winkellagensensor zur Messung einer absoluten Winkellage einer Drehachse eines Lenkrades in einem Fahrzeug. Ein Sensor mit Fotosensoren, wie beispielsweise Fotodioden, dient der Messung der Lichtintensität von empfangenen Lichtstrahlen, die in Abhängigkeit einer Rotation einer Rotationsscheibe oszillieren. Die Polarisation, welche über eine räumlich feste lineare Polarisationseinheit und eine rotierende Polarisationseinheit induziert wird, ist für jeden Lichtstrahl verschieden. Der aktuelle Wert einer absoluten Winkellage des drehenden Teils wird über eine Berechnungseinrichtung aus den Messdaten der Fotosensoren und über eine Leseeinrichtung eines absoluten optischen Winkelencoders eindeutig berechnet.

Die US-A-4,555,625 betrifft eine Einrichtung zur Positionserkennung mit einer Vielzahl von lichtempfindlichen Sensoren, die an einem Rahmenglied befestigt sind. Entsprechend der Anzahl der lichtempfindlichen Sensoren ist eine Vielzahl von Lichtquellen vorgesehen, die mit den Sensoren zusammenwirken und an dem Rahmenglied befestigt sind. Es ist eine Vielzahl von trommelförmigen Masken zwischen den Lichtquellen und den lichtempfindlichen Sensoren angeordnet, wobei zumindest eine der Masken einen kleineren Umfang als eine andere Maske aufweist. Die Masken sind starr an einem Arm befestigt, der relativ zu dem Rahmenglied drehbeweglich ist, wobei die Masken das Zusammenwirken zwischen Lichtquellen und lichtempfindlichen Sensoren als Funktion einer Rotationsbewegung des Armes unterbrechen. Dabei sind zumindest zwei trommelförmige Masken derart zueinander positioniert, dass sich eine erste Maske zumindest teilweise in der zweiten Maske befindet.

Die US-A-5,376,785 betrifft einen optischen Verschiebungssensor, der ein längliches Hohlgehäuse und eine darin befindliche zentrale Kammer aufweist. Eine Trennwand unterteilt die zentrale Kammer in einen ersten Abschnitt und einen zweiten Abschnitt, wobei die Trennwand eine zentrale Öffnung aufweist. Ein Licht aussendendes Mittel und ein dem Licht aussendenden Mittel gegenüberliegendes Licht empfangendes Mittel sind in dem ersten Abschnitt der zentralen Kammer angeordnet. Ein axial bewegliches Licht blockierendes Mittel erstreckt sich zwischen dem Licht aussendenden Mittel und dem Licht empfangenden Mittel, um das vom Licht aussendenden Mittel ausgehende Licht zumindest teilweise zu blockieren.

Die GB 1 498 409 A betrifft ein Gerät zur Erzeugung elektrischer Signale, die in Abhängigkeit der Winkellage eines Rotationsgliedes variieren. Das Gerät weist eine Platte zur Rotation mit dem Rotationsglied auf, wobei eine Hälfte der Platte aus einem Licht durchlässigen, polarisierenden Material und der übrige Teil aus einem Licht abschirmenden Material besteht. Positionsfeste Analysevorrichtungen sind räumlich um die Rotationsachse der Platte angeordnet. Das Gerät weist Beleuchtungsmittel und entsprechende fotoelektrische Einrichtungen für jede der positionsfesten Analysevorrichtungen auf. Die Bauteile sind derart angeordnet, dass der vom Beleuchtungsmittel zur fotoelektrischen Vorrichtung führende Lichtweg die positionsfesten Analyseeinrichtungen und die Platte beinhaltet. Des Weiteren sind Mittel vorgesehen, um die Ausgangssignale der fotoelektrischen Vorrichtungen zu kombinieren. Die Polarisationsrichtungen des polarisierenden Materials der Platte und der Analyseeinrichtungen bewirken, dass das Ausgangssignal des kombinierenden Mittels sinusartig mit der Rotation der Platte variiert.

Die DE 10 2006 045 138 A1 betrifft eine Vorrichtung und ein Verfahren zur Vermessung des Wirbelsäulenverlaufes mit einer Lichtquelle, die Licht mit einer konstanten Lichtleistung in ein erstes Ende eines Lichtleiters einspeist. An dem zweiten Ende des Lichtleiters ist ein die ankommende Lichtleistung messender Empfänger angeordnet, der mit einer Auswerteeinheit verbunden ist. Der Lichtleiter weist eine biegesensitive Zone auf, aus der Licht aus dem Lichtleiter aufgrund einer Störung im Kern-Mantel-Übergang bei einer Biegung nach außen dringt. Die veränderte Lichtleistung an dem Empfänger lässt Rückschlüsse auf eine Verlaufsänderung des Lichtleiters zu.

Aufgabe der vorliegenden Erfindung ist es, eine Vorrichtung und ein Verfahren bereitzustellen, mit denen kostengünstig und zuverlässig eine unmittelbare Messwerterfassung möglich ist.

Erfindungsgemäß wird diese Aufgabe durch eine Vorrichtung mit den Merkmalen des Anspruchs 1 und ein Verfahren mit den Merkmalen des Anspruchs 13 gelöst. Vorteilhafte Ausgestaltungen und Weiterbildungen der Erfindung sind in den Unteransprüchen, der Beschreibung und den Figuren offenbart.

Die Vorrichtung zur Bestimmung von Relativverlagerungen von Körperteilen oder Körperbereichen mit einem Sender und einem dem Sender zugeordneten Sensor sieht vor, dass zwischen dem Sender und dem Sensor zumindest eine relativ zu dem Sensor und/oder dem Sender verlagerbare Abschattungseinrichtung angeordnet ist, die an zumindest einem Körperteil oder Körperbereich festlegbar ist. Der Sensor und/oder der Sender sind an dem Körper festlegbar ausgebildet. Durch das Festlegen der Abschattungseinrichtung an einem Körperteil oder einem Körperbereich ist es möglich, eine Relativbewegung in diesem Körperbereich oder zwischen zwei Körperteilen einfach und mit einer hohen Genauigkeit und Ortsauflösung festzustellen. So ist es insbesondere möglich, eine Verdrehung der Wirbelsäule über deren gesamte Länge durch die Festlegung der Abschattungseinrichtung an einem Ende und die Anordnung des Sensors an dem anderen Ende der Wirbelsäule zu detektieren. Ebenso ist es möglich, durch die beliebige Positionierung des Sensors entlang der Abschattungseinrichtung eine präzise Festlegung der Messstelle zu erreichen, so dass keine Modellbildung mehr notwendig ist, um die Relativverlagerung zu bestimmen. Als Abschattungseinrichtung wird auch die gerichtete Abstrahlung von Signalen, beispielsweise Licht, Strahlung, elektromagnetischen Wellen oder magnetische Muster, in einer Richtung verstanden, so dass beispielsweise eine nur in eine Richtung abstrahlende Lichtquelle auf der der Abstrahlrichtung abgewandten Seite kein oder nur wenig Licht emittiert, was den gleichen Effekt hat, als wenn eine allseitig streuende Lichtquelle bereichsweise durch eine Blende oder dergleichen abgedunkelt wird. Entsprechendes gilt für alternative Signalformen, bei denen keine Lichtwellen verwendet werden. Der Grundkörper ist biegsam ausgebildet, so dass er den vielfältigen Bewegungsmöglichkeiten des Körpers folgen und mit nur einem Grundkörper eine Vielzahl von Messstellen bedient werden kann.

Die Abschattungseinrichtung kann zwischen dem Sensor und dem auf den Sensor gerichteten Sender angeordnet sein. Dies ist insbesondere dann der Fall, wenn der Sender oder die Austrittsstelle des Senders gegenüber dem Sensor liegt und die Abschattungseinrichtung zwischen dem Sender bzw. Austrittsstelle des Senders und dem Sensor angeordnet ist. Die Abschattungseinrichtung wird also in Richtung auf den Sensor durchsendet, durchdrungen, durchleuchtet oder angeleuchtet, wodurch es möglich ist, eine hohe Signalintensität in Richtung auf den Sensor zu richten.

Die Abschattungseinrichtung ist an dem als Signalleiter ausgebildeten, biegsamen Grundkörper angeordnet oder ausgebildet, wobei der Sender neben dem Signalleiter angeordnet ist und diesen anstrahlt oder so dem Signalleiter zugeordnet ist, dass der Sender das Signal oder die Signale in Längserstreckung des Signalleiters in diesen einspeist. Die Abschattungseinrichtung wird von dem Signal in Richtung auf den Sensor durchsendet, durchdrungen, durchleuchtet oder angeleuchtet. Durch die innerhalb des Signalleiters vorhandene Reflexion können über die gesamte Länge des Signalleiters, z.B. Lichtleiters, Signale, Strahlung, elektromagnetische Wellen oder Licht aus dem Signalleiter radial nach außen dringen. Durch Einbau entsprechender Elemente, z.B. optischer Elemente, wie Spiegel, Prismen oder dergleichen ist es möglich, eine gezielte Auslenkung des Signals in bestimmten Bereichen oder Richtungen vorzusehen. Wird ein punktuell oder streifenförmig gerichteter Strahl in eine nach radial außen gerichtete Richtung abgelenkt, ist der übrige Umfang des Signalleiters als Abschattungseinrichtung anzusehen, da in diesem Bereich weniger Signal nach außen dringt als in dem Bereich, in dem das Signal aus dem Signalleiter ausgeleitet wird. Die Einspeisung des Signals in Längserstreckung des Signalleiters hat den Vorteil, dass über die gesamte Länge des Signalleiters nur ein Sender vorgesehen sein muss und viele Sensoren entlang des Signalleiters angeordnet sein können, um die jeweilige Verlagerung zu detektieren.

Eine Variante der Erfindung sieht vor, dass der Sender als eine Lichtquelle, Magnet, Strahler oder elektromagnetische Wellen emittierender Sender ausgebildet ist. Der Sender ist dabei korrespondierend zu dem Sensor ausgebildet und sendet Signale oder ein Signal, das von dem Sensor erfasst werden kann. Das Signal kann dabei direkt in Richtung auf den Sensor gerichtet abgestrahlt oder über einen Leiter oder mehrere Leiter in Richtung auf den Sensor geleitet und auf diesen ausgerichtet werden.

Es ist möglich, dass mehrere Abschattungseinrichtungen in Verlagerungsrichtung der Abschattungseinrichtung hintereinander angeordnet sind. Ist überwiegend eine Verdrehung der Abschattungseinrichtung vorgesehen, können mehrere Abschattungseinrichtungen in Rotationsrichtung um den Umfang beispielsweise eines Grundkörpers herum angeordnet sein. Wird eine Längenveränderung oder eine Beugung, die mit einer Längenveränderung einhergeht, zu messen sein, können in Längserstreckung entlang der Verlagerungsrichtung mehrere Abschattungseinrichtungen hintereinander angeordnet sein.

Die Abschattungseinrichtung kann als eine auf einem Grundkörper aufgebrachte Beschichtung oder als ein Bereich des Grundkörpers mit abweichender Signaldurchlässigkeit, beispielsweise Lichtdurchlässigkeit oder Lichtabstrahlung, ausgebildet sein. Es ist grundsätzlich auch möglich, dass die Abschattungseinrichtung aus einem für das Signalmedium undurchlässigen, beispielsweise lichtundurchlässigen Element besteht, das eine nicht rotationssymmetrische Struktur aufweist oder Abflachungen oder Konturmuster aufweist, mit denen es möglich ist, eine Relativverlagerung zu detektieren. Bei einer Abflachung ergibt sich die Detektion aufgrund der vergrößerten oder verringerten Abschattung des Sensors bei einer Verdrehung; bei einer Verschiebung ist eine Konturierung in Gestalt eines Musters, beispielsweise durch Vorsprünge in Längserstreckung an der Außenseite, ein möglicher Indikator für eine Relativverlagerung. Sofern die Abschattungseinrichtung durchstrahlt oder durchleuchtet wird, sind beispielsweise Beschichtungen auf einem strahlungs- oder lichtdurchlässigen Grundkörper möglich und vorgesehen, die vorteilhafterweise an der Außenseite aufgebracht sind. Diese Beschichtungen können in Streifenform in Längsrichtung oder in Umfangsrichtung angeordnet sein, um bei Rotation oder Längsverlagerung die Einfallrate auf den Sensor zu variieren. Ebenfalls ist es möglich, dass zumindest ein Bereich des Grundkörpers mit einer abweichenden Signaldurchlässigkeit oder Signalabstrahlung ausgebildet ist, also beispielsweise durch Aufrauung oder durch Veränderung des Brechungsindexes eine Streuung oder Absorption des einfallenden oder durchgeleiteten Signals, z.B. Lichts bewirkt, so dass in Abhängigkeit von der Position des Grundkörpers unterschiedliche Signalintensitäten auf den Sensor auftreffen. Es ist ebenfalls möglich und vorgesehen, dass die Abschattungseinrichtung als Filter, z.B. Polarisationsfilter ausgebildet ist. Durch die Relativverlagerung des Filters wird der Transmissionsgrad verändert, was zu unterschiedlichen Intensitäten auf dem Sensor führt. Vorteilhafterweise gibt der Sender polarisiertes Licht ab, um den Effekt zu verstärken. Der Abschattungsbereich kann auch als ein Bereich mit einem abweichenden magnetischen Fluss, einer abweichenden Feldlinienausrichtung oder Feldliniendichte ausgebildet sein, so dass über einen magnetosensitiven Sensor eine Veränderung der Lage detektiert werden kann.

Der Grundkörper kann einen im Wesentlichen runden Querschnitt aufweisen, was insbesondere bei Abschattungseinrichtungen in Gestalt von Beschichtungen oder Streifenmustern vorteilhaft für die Ermittlung von Verdrehungen, insbesondere einer Torsion einer Wirbelsäule, ist. Wenn der Grundkörper langgestreckt ausgebildet ist und das Signal, z.B. Licht senkrecht zu der Längserstreckung des Grundkörpers in Richtung auf den Sensor fällt, wird der Grundkörper durchdrungen durchleuchtet oder Licht wird auf den lichtleitenden Grundkörper heraus in Richtung auf den Sensor geleitet. Bei einem runden Querschnitt des Grundkörpers wird der Grundkörper mit der Abschattungseinrichtung als Drehachse ausgebildet, die selbst eine optische Blende oder Abschattung bewirkt, wodurch sich ein sehr kleiner und kostengünstiger Aufbau erreichen lässt. Entsprechendes gilt für einen nicht lichtdurchlässigen Grundkörper mit einem nicht rotationssymmetrischen Querschnitt oder einem in Längsrichtung verlaufenden Muster in der Kontur.

Es können mehrere Messstellen an einer Abschattungseinrichtung um oder entlang einer Längsachse der Abschattungseinrichtung möglich sein, die wiederum beliebig flexibel ausgebildet sein kann, wobei lediglich eine ausreichende Torsionssteifigkeit gewährleistet sein muss, um eine Verdrehung des Körpers, an dem der Grundkörper festgelegt ist, detektieren zu können. Bei einer Längenmessung ist eine Längenveränderlichkeit vorteilhafterweise nicht gegeben, was bei einem flexiblen Grundkörper, beispielsweise durch Einarbeitung eines flexiblen, jedoch unelastischen Elementes, beispielsweise eines hochfesten Fasermaterials, erreicht werden kann. Zur Ermittlung der Verdrehung ist es vorteilhaft, wenn die Abschattungseinrichtung an einem Ende torsionsfest an einem Körperteil oder Körperbereich festlegbar und an dem anderen Ende frei drehbar ist. Neben der freien Drehbarkeit kann an dem anderen Ende auch eine Verschieblichkeit relativ zu der Sensoreinrichtung vorgesehen sein, so dass auch eine Längenveränderung erfasst werden kann. Befindet sich der Sender gegenüber dem Sensor, sind diese vorteilhafterweise ortsfest zueinander festgelegt, beispielsweise auf einer aufklebbaren oder auf der Kleidung aufklettbaren oder anderweitig befestigbaren Trägereinrichtung, erfolgt der Lichtaustritt in Richtung auf den Sensor durch einen Grundkörper, an dem die Abschattungseinrichtung angeordnet oder ausgebildet ist, entfällt die starre Zuordnung des Sensors zu einem Sender.

Der Abschattungseinrichtung kann zumindest eine Einrichtung zur Festlegung an einem Körperteil oder Körperbereich zugeordnet sein, an der zumindest ein Sensor befestigt ist und an der die Abschattungseinrichtung relativ zu dem Sensor verlagerbar gelagert ist. Dadurch wird die Zuordnung des Sensors zu einer bestimmten Stelle an dem Körper sichergestellt und eine Führung der Abschattungseinrichtung sichergestellt, so dass bei einer Verlagerung der Abschattungseinrichtung die räumliche Zuordnung, abgesehen von einem oder zwei Freiheitsgraden, erhalten bleibt. Die Einrichtung zur Festlegung an einem Körperteil kann Führungseinrichtungen aufweisen, in denen die Abschattungseinrichtung verdrehbar und/oder verlagerbar geführt ist. Diese Einrichtungen können als Muffen, Hülsen oder dergleichen ausgebildet sein. An den Einrichtungen sind Sensoren oder Aufnehmer für Leitungen, die das Signal zu den Sensoren leitet, angeordnet, so dass durch eine Relativverlagerung der Abschattungseinrichtung zu den Einrichtungen und damit zu den Sensoren oder den Aufnehmern die Verdrehung oder Verschiebung detektiert werden kann.

Der Sensor ist vorteilhafterweise als eine Einrichtung ausgebildet, die die Signalintensität in einem oder mehreren Frequenzbereichen des Signal, z.B. Licht, misst. Als Sensoren sind Infrarotdetektoren ebenso vorgesehen wie Fotodioden oder Fototransistoren. Eine Beschränkung auf das sichtbare Lichtspektrum ist nicht vorgesehen. Es können mehrere Sensoren in Längserstreckung der Abschattungseinrichtung hintereinander angeordnet sein. Dem Sensor kann eine Auswerteeinrichtung zugeordnet sein, über die ermittelt wird, in welche Richtung und in welchem Umfang eine Relativverlagerung stattfindet. Vor den Sensor und gegebenenfalls vor dem Sender kann eine Blende angeordnet sein, um die Erfassung von Streusignalen zu vermeiden.

Das Verfahren zur Messung einer Relativverlagerung von Körperteilen oder Körperbereichen zueinander sieht vor, dass eine Abschattungseinrichtung an einer Stelle ortfest an einem ersten Körperteil oder Körperbereich festgelegt wird, dass ein Signal in Richtung auf einen Sensor gesendet und die an einem als biegsamen Leiter ausgebildete Abschattungseinrichtung von dem Signal in Richtung auf den Sensor durchsendet, durchdrungen, durchleuchtet oder angeleuchtet und aufgrund einer Relativverlagerung von Körperteilen oder Körperbereichen verlagert wird. Ein sich aufgrund der Verlagerung der Abschattungseinrichtung verändernder Signaleinfall auf den Sensor wird erfasst und in einer Auswerteeinrichtung ausgewertet. Je nach Veränderung des Signaleinfalls ergibt sich ein unterschiedliches Signal für den Sensor, so dass je nach Möglichkeit der Relativbewegung von Signal zum Sensor die entsprechende Bewegung detektiert wird. Ist lediglich eine Rotation möglich, wird eine Rotation detektiert, ist eine Längsverlagerung möglich, wird eine entsprechende Längsverlagerung detektiert. Grundsätzlich ist es auch möglich, eine kombinierte Verlagerung durch Kombination mehrerer Abschattungseinrichtungen oder Sensoren zu erfassen. Voraussetzung ist, dass der Sensor oder ein Aufnehmer an dem Körperteil möglichst ortsfest angeordnet ist, beispielsweise aufgeklebt oder durch ein Kleidungsstück an der gewünschten Position gehalten wird, während die Abschattungseinrichtung relativ zu dem Sensor oder Aufnehmer verlagerbar ist.

Die Abschattungseinrichtung kann als langgestreckter Grundkörper ausgebildet sein, wobei ein Ende des Grundkörpers torsionsfest festgelegt wird und zumindest zwei versetzt zueinander angeordnete Sensoren neben der Abschattungseinrichtung angeordnet sind, so dass die Drehrichtung durch Auswertung der Phasenverschiebung der Sensorsignale bestimmt werden kann. Neben dem Umfang der Bewegung wird somit auch die Bewegungsrichtung detektiert. Alternativ zu der Bestimmung der Drehrichtung kann durch Anordnung zweier Sensoren hintereinander in Verlagerungsrichtung auch eine Bestimmung sowohl der Längsverlagerung als auch der Verlagerungsrichtung erfolgen.

Das Signal kann in den als Signalleiter ausgebildeten, biegsamen Grundkörper entlang dessen Längserstreckung eingespeist und das Signal in Richtung auf den Sensor aus dem Signalleiter ausgekoppelt werden, wobei an dem Signalleiter zumindest eine Abschattungseinrichtung angeordnet oder ausgebildet ist.

Die vorbeschriebene Vorrichtung dient insbesondere zur Ausführung des ebenfalls vorbeschriebenen Verfahrens. Durch die beanspruchte Erfindung ist es insbesondere möglich, eine Messung der Wirbelsäulentorsion bzw. Rotation durch das Auftragen oder Verbinden eines Sensors mit einem textilen Kleidungsstück durchzuführen. Ebenfalls besteht die Möglichkeit, eine Längenänderung, beispielsweise durch Verformung des Textils zu erfassen. Der Aufbau der Erfindung ermöglicht eine raumsparende und kostengünstige Lösung, die unempfindlich die meisten Umwelteinflüsse ist. Mehrere Messstellen entlang einer Achse sind möglich, wobei die Achse beliebig flexibel sein kann, eine Elastizität bezüglich der zu messenden Bewegung jedoch nicht möglich ist.

Nachfolgend werden Ausführungsbeispiele der Erfindung anhand der beigefügten Figuren näher erläutert. Es zeigen:
- Figur 1 -: einen prinzipiellen Aufbau einer Messvorrichtung;
- Figur 2 -: einen schematischen Intensitätsverlauf eines Messsignals;
- Figur 3 -: eine Variante der Figur 1;
- Figur 4 -: eine weitere Variante der Figur 1;
- Figur 5 -: eine Vorrichtung zur Messung einer Relativverschiebung;
- Figur 6 -: ein Ausführungsbeispiel der Erfindung im angelegten Zustand;
- Figur 7 -: eine Vorrichtung gemäß Figur 6 im tordierten Zustand sowie
- Figur 8 -: eine Vorrichtung gemäß Figur 3 mit Lichtleitern zu einer Lichtquelle und einem Sensor.

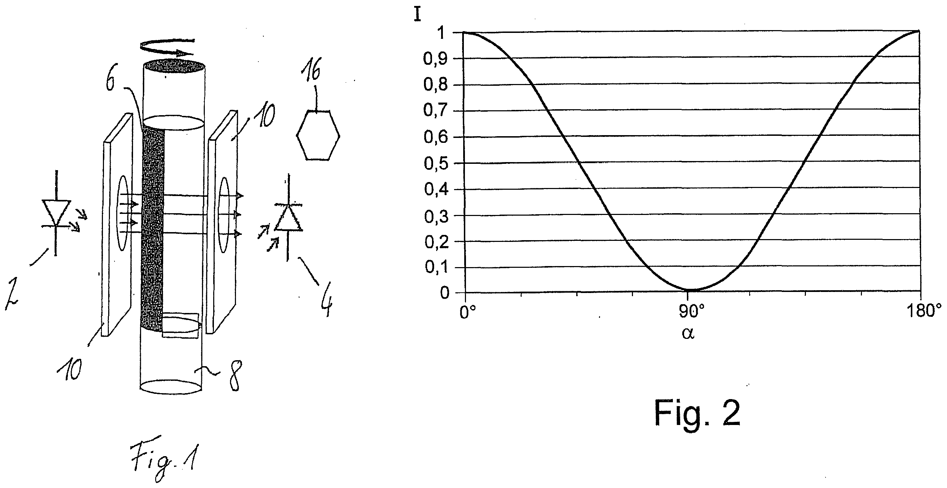

In der Figur 1 ist in einer schematischen Darstellung eine Vorrichtung zur Bestimmung von Relativverlagerungen von Körperteilen und Körperbereichen mit einer Lichtquelle als Sender 2 dargestellt, die durch zwei Blenden Licht in Richtung auf einen Sensor 4 sendet. Der Sender 2 ist als Licht emittierende Diode (LED) ausgebildet. Der Sensor 4 ist als Fotodiode ausgebildet. Zwischen dem Sender 2 und dem Sensor 4 ist eine Abschattungseinrichtung 6 angeordnet, die auf einem langgestreckten Grundkörper 8 befestigt ist. Die Abschattungseinrichtung 6 im dargestellten Ausführungsbeispiel ist als eine lichtundurchlässige Beschichtung auf der Außenseite aufgebracht, alternative Abschattungseinrichtungen sind möglich. Beispielsweise kann der Grundkörper 8 aus einem lichtundurchlässigen Material mit einer Abflachung an einer Seite des Umfanges angeordnet sein, so dass durch Verdrehen des Grundkörpers 8 um seine Längsachse der Sensor 4 mit einer unterschiedlichen Strahlungsintensität beaufschlagt wird. Die Drehung um die Längsachse des Grundkörpers 8 ist durch den Pfeil dargestellt. Alternierende Drehbewegungen können möglich sein. Im dargestellten Ausführungsbeispiel ist der Grundkörper 8 grundsätzlich lichtdurchlässig, so dass bis auf den Bereich, der mit einer Abschattungseinrichtung 6 versehen ist, das Licht von dem Sender 2 im Wesentlichen ungestört durch die Blenden 10 auf den Sensor 4 treffen kann. Von dem Sensor 4 wird das empfangene Signal zu einer Auswerteeinrichtung 16 geleitet, wo die Auswertung des Signals der Lichtintensität erfolgt.

In der Figur 2 ist schematisch die Intensität des Lichteinfalls über den Drehwinkel *α* aufgezeichnet. In einer Ausgangsposition von 0° ist eine maximale Lichtintensität gegeben. Bei einer Verdrehung des Grundkörpers 8 und damit auch der Abschattungseinrichtung 6 wird die Intensität je zunehmend verringert, bis sie bei einer Verdrehung um 90° 0 ist, was bedeutet, dass die Blenden 10 vollständig von der Abschattungseinrichtung 6 überdeckt sind, so dass kein Licht von dem Sender 2 zum Sensor 4 gelangt. Wird die Abschattungseinrichtung 6 weiter verdreht, nimmt die Lichtintensität I wieder zu, bis sie bei einer Verdrehung um 180°, wenn die Blenden 10 wieder vollständig freigegeben sind, auf dem Intensitätsmaximum wieder angelangt ist. Je nach Größe der Blenden 10 sowie der Abschattungseinrichtung 6 kann der Bereich der vollständigen Abschattung über einen anderen Drehbereich erfolgen, ebenfalls kann es sein, dass die Abschattung nicht vollständig erfolgt, so dass bei Erreichen eines Intensitätsminimums der Sensor 4 weiterhin Licht detektiert. Auch kann es möglich sein, dass Bereich der vollständigen Abschattung der Blenden 10 nach einer geringeren Verschwenkung als um 90° erfolgt, so dass eine vollständige Abschattung des Lichteinfalls auf den Sensor 4 früher erfolgt.

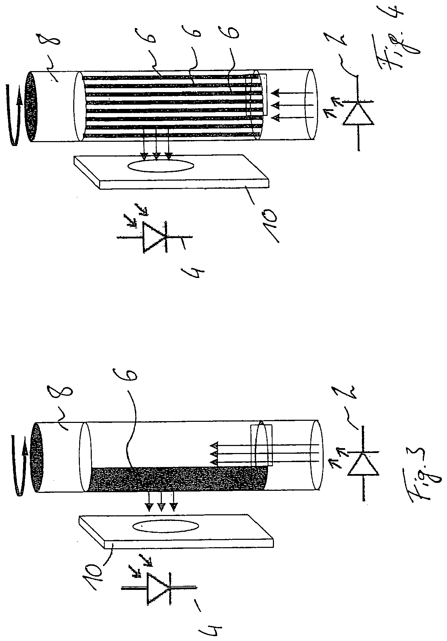

Eine Variante der Erfindung ist in der Figur 3 dargestellt, bei der statt einer Durchleuchtung des durchsichtigen Grundkörpers 8 in Richtung auf den Sensor 4 das Licht des Senders 2 in Längsrichtung des Grundkörpers in diesen eingespeist wird. Der Grundkörper 8 fungiert damit als Lichtleiter, in dem das Licht sich in Längserstreckung fortbewegt. Durch die Reflexion auf der Innenseite des Grundkörpers 8 wird ein Teil des Lichtes radial nach außen abgelenkt, so dass eine Lichtstrahlung durch die Blende 10 auf den Sensor 4 fällt. Die radial nach außen dringende Strahlung wird durch die Abschattungseinrichtung 6, beispielsweise eine reflektierende Beschichtung, zurückgehalten, so dass das in der Figur 2 dargestellte Intensitätsmuster bei einer Verdrehung des Grundkörpers 8 relativ zu dem Sensor 4 auftritt. Grundsätzlich ist es auch möglich, dass statt einer relativ schmalen Abschattungseinrichtung 6 nur ein relativ schmales Lichtfenster in der Abschattungseinrichtung 6 vorhanden ist, um einen Lichtstreifen auf den Sensor 4 zu werfen. Eine entsprechende Intensitätsverteilung wird dann auch erreicht, allerdings auf einem geringeren Niveau.

Eine weitere Variante der Vorrichtung ist in der Figur 4 gezeigt, bei der eine Vielzahl von Abschattungseinrichtungen 6 in Gestalt von lichtundurchlässigen Streifen um den Umfang des Grundkörpers 8 herum angeordnet sind. Die Lichteinkoppelung erfolgt an einem stirnseitigen Ende des Grundkörpers 8, die Auskopplung des Lichtes in Richtung auf den Sensor 4 kann durch optische Einbauten innerhalb des Grundkörpers 8, beispielsweise durch Prismen oder Reflektionseinrichtungen verstärkt oder bewirkt werden. Durch die Anordnung mehrerer Abschattungseinrichtungen 6 in Umfangsrichtung, im Falle der Drehung um die Längsachse des Grundkörpers 8, also in Verlagerungsrichtung hintereinander, können Drehrichtung und Verdrehung leicht ermittelt werden, insbesondere wenn die Abschattungseinrichtungen 6 nicht äquidistant zueinander an dem Grundkörper 8 angeordnet sind.

Werden mehrere Sensoren 6 in Verlagerungsrichtung hintereinander angeordnet, kann durch Auswertung einer Phasenverschiebung neben dem Drehwinkel auch die Drehrichtung ermittelt werden.

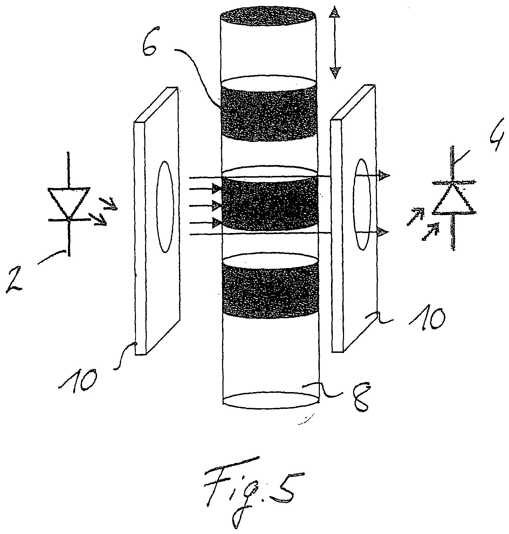

In der Figur 5 ist eine Variante der Erfindung darstellt, bei der statt einer Verdrehung eine Längsverschiebung des Grundkörpers 8 und damit auch der fest darauf angeordneten Abschattungseinrichtungen 6 erfolgt. Auch hier liegen der Sender 2 und der Sensor 4 einander gegenüber, wobei zwei Blenden 10 zwischen Lichtquelle 2 und Sensor 4 angeordnet sind, zwischen denen der Grundkörper 8 mit den Abschattungseinrichtungen 6 angeordnet ist. Die Längsverschieblichkeit ist durch den Doppelpfeil angedeutet. Die Abschattungseinrichtungen 6 sind als Ringe in einem Abstand zueinander angeordnet, so dass sich lichtundurchlässige Bereiche mit lichtdurchlässigen Bereichen abwechseln. Im dargestellten Beispiel ist die zwischen den Blenden 10 angeordnete Abschattungseinrichtung 6 so bemessen, dass die Blenden 10 nicht vollständig geschlossen werden, so dass ein Teil der Lichtstrahlen von dem Sender 2 auf den Sensor 4 trifft. Wird der Grundkörper 8 mit der Abschattungseinrichtung 6 nach oben oder unten bewegt, erhöht sich die Lichtintensität um den Anteil, um den die nicht abgeschattete Fläche der Blenden 10 vergrößert wird. Werden mehrere Sensoren 4 in Verlagerungsrichtung hintereinander angeordnet, kann aus der Phasenverschiebung neben dem Verlagerungsweg auch die Verlagerungsrichtung bestimmt werden.

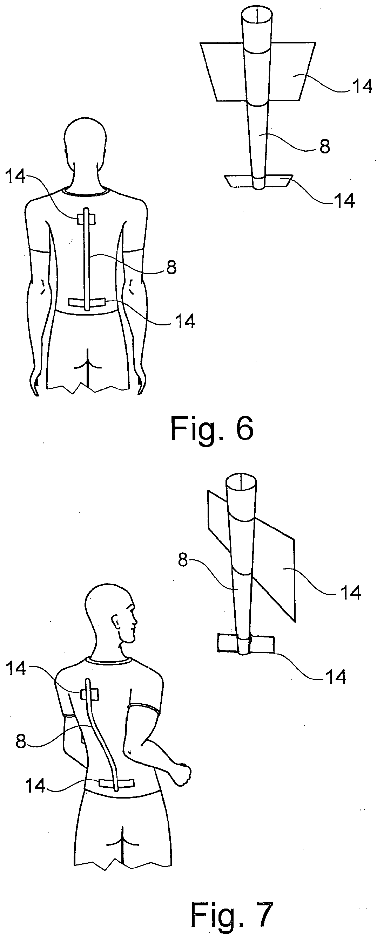

Ein angewandtes Ausführungsbeispiel ist in den Figuren 6 und 7 gezeigt, die Figur 6 zeigt eine Person mit einem eng anliegenden Kleidungsstück, auf dessen Oberfläche eine Vorrichtung zur Bestimmung der Wirbelsäulenverlagerung befestigt ist. Ein Grundkörper 8 ist im Lendenwirbelbereich über eine Einrichtung 14 zur Festlegung an dem Kleidungsstück befestigt. Die Befestigung erfolgt beispielsweise über ein Klebeband, so dass eine torsionssteife Befestigung im Lendenwirbelbereich erfolgt. Eine zweite Einrichtung 14 zur Festlegung an einem Körperteil ist im oberen Brustwirbelbereich vorgesehen. In der oberen Darstellung der Figur 6 ist die Ausrichtung sowohl des Grundkörpers 8 als auch der Einrichtungen 14 zur Festlegung an einem Körperteil in einer Position gezeigt, in der die Schultern im Wesentlichen parallel zum Becken stehen. Diese Ausrichtung wird durch den senkrechten Strich an dem oberen Ende des Grundkörpers 8 verdeutlicht. Die Festlegung kann auch direkt an dem Körper auf der Haut erfolgen.

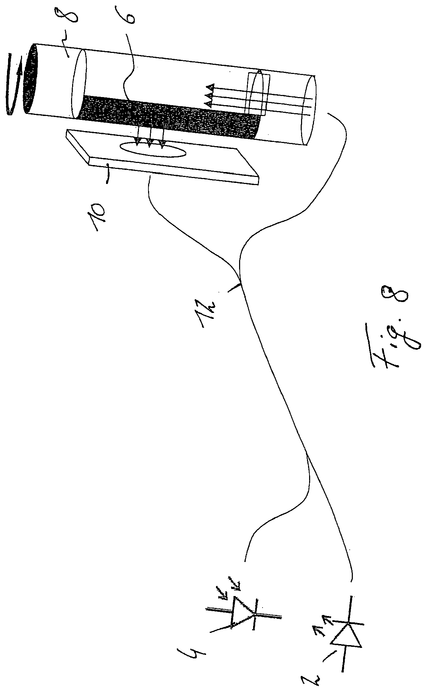

In der Figur 7 ist eine tordierte Stellung der Wirbelsäule der Person gezeigt. Der Oberkörper hat sich dabei von oben gesehen in Uhrzeigerrichtung relativ zu dem Becken verdreht, so dass sich eine Torsion der Wirbelsäule in Uhrzeigerrichtung ergeben hat. In der oberen Darstellung der Figur 7 ist zu erkennen, dass ausgehend von der unteren Einrichtung 14 zur Festlegung der Grundkörper 8 starr geblieben ist, also sich nicht relativ zu dem Becken und der unteren Einrichtung 14 verdreht hat, die obere Einrichtung 14 zur Festlegung an dem Körperteil jedoch sich in dem Maße relativ zu dem Grundkörper 8 verdreht hat, wie sich der Oberkörper der Person verdreht hat. Ist nun an der oberen Einrichtung 14 zur Festlegung an einem Körperteil oder einem Körperbereich ein Sensor 4 angeordnet und gegebenenfalls auch eine Lichtquelle 2 auf der gegenüberliegenden Seite, kann durch die Relativverdrehung der oberen Einrichtung 14 und damit auch des Sensors eine Bestimmung des Verdrehwinkels entlang der Wirbelsäule vorgenommen werden. Sind mehrere Sensoren und Abschattungseinrichtungen entlang der Längserstreckung des Grundkörpers 8 angeordnet und erfolgt eine Lichteinspeisung beispielsweise an einer unteren Stirnseite des Grundkörpers 8, kann mit einer Lichtquelle eine hoch aufgelöste Messung der einzelnen Verdrehwinkel entlang der Wirbelsäule mit mehreren Sensoren erfolgen. Der Grundkörper 8 ist zusammen mit der Abschattungseinrichtung 6 relativ verlagerbar zu der oberen Einrichtung 14 angeordnet, im dargestellten Ausführungsbeispiel verdrehbar und längsverschieblich angeordnet, so dass auch bei einer Beugebewegung nach vorn und nach hinten eine Relativverlagerung zu der Einrichtung 14 zum Festlegen erfolgen kann. An der unteren Einrichtung 14 ist der Grundkörper 8 insbesondere torsionsfest und unverschieblich festgelegt.
Eine Variante der Erfindung ist in der Figur 8 gezeigt, bei der über Lichtleiter 12 Licht auf einer Seite von dem Sender 2 in den Grundkörper 8 eingespeist und von diesem in Richtung auf eine Blende 10 geworfen wird. Je nach Stellung der Abschattungseinrichtung 6 oder der Abschattungseinrichtungen wird eine mehr oder weniger große Lichtintensität durch die Blende 10 hindurch fallen, die wiederum über einen Lichtleiter 12 aufgenommen und zum Sensor 4 geleitet wird. Dadurch ist es möglich, dass sowohl der Sender 2 als auch der Sensor 4 entfernt von derjenigen Stelle angeordnet werden kann, an der die Relativverlagerung der Körperbereiche zueinander erfolgt. Der Grundkörper 8 muss dann lediglich in einer Führung, beispielsweise einer Hülse, geführt werden, in der oder an der auch der rückführende Lichtleiter 12 zur Fotodiode angeordnet ist. Dadurch kann die Vorrichtung sehr klein gehalten werden, der Sender 2, der Sensor 4 sowie die Auswerteeinrichtung 16 können dann an einem anderen Ort am Körper angebracht werden, der gegebenenfalls weniger exponiert ist, so dass die gesamte Vorrichtung leicht und unauffällig zu tragen ist. Die Auswerteeinrichtung 16 kann beispielsweise in einer Tasche angeordnet sein, die an einem Kleidungsstück befestigt werden kann.

Die Erfindung wurde vorstehend anhand eines Senders 2 in Gestalt einer Lichtquelle beschrieben. Die Funktionsweise ändert sich jedoch nicht bei der Verwendung anderer Signalformen, also elektromagnetischer Wellen, induktive Energieübertragung, magnetische Felder und dergleichen. Lediglich die Sensoren und die Übertragungsmedien werden an die Signalform angepasst.

## Patentansprüche

1. Vorrichtung zur Bestimmung von Relativverlagerungen von Körperteilen oder Körperbereichen zueinander mit einem Sender (2) und einem dem Sender (2) zugeordneten Sensor (4), wobei zwischen dem Sender (2) und dem Sensor (4) zumindest eine relativ zu dem Sensor (4) und/oder dem Sender (2) verlagerbare und an einem als Leiter ausgebildeten Grundkörper (8) angeordnete oder ausgebildete Abschattungseinrichtung (6) angeordnet ist, die an zumindest einem Körperteil oder Körperbereich festlegbar ist und der Sender (2) neben dem Leiter angeordnet ist und diesen anstrahlt oder das Signal in Längserstreckung des Leiters in diesen einspeist, wobei der Grundkörper (8) biegsam ausgebildet ist und die Abschattungseinrichtung (6) von dem Signal in Richtung auf den Sensor (4) durchsendet, durchdrungen, durchleuchtet oder angeleuchtet wird.

2. Vorrichtung nach Anspruch 1, **dadurch gekennzeichnet, dass** der Sender (2) als eine Lichtquelle, Magnet, Strahler oder elektromagnetische Wellen emittierender Sender ausgebildet ist.

3. Vorrichtung nach Anspruch 1 oder 2, **dadurch gekennzeichnet, dass** die Abschattungseinrichtung (6) zwischen dem Sensor (4) und dem auf den Sensor (4) gerichteten Sender (2) angeordnet ist.

4. Vorrichtung nach einem der voranstehenden Ansprüche, **dadurch gekennzeichnet, dass** mehrere Abschattungseinrichtungen (6) in Verlagerungsrichtung hintereinander angeordnet sind.

5. Vorrichtung nach einem der voranstehenden Ansprüche, **dadurch gekennzeichnet, dass** die Abschattungseinrichtung (6) als eine auf einem Grundkörper (8) aufgebrachte Beschichtung, als Polarisationsfilter oder als ein Bereich des Grundkörpers (8) mit abweichender Durchlässigkeit oder Abstrahlung, abweichender Feldlinienausrichtung oder abweichendem magnetischen Fluss ausgebildet ist.

6. Vorrichtung nach Anspruch 5, **dadurch gekennzeichnet, dass** der Grundkörper (8) einen im Wesentlichen runden Querschnitt aufweist.

7. Vorrichtung nach Anspruch 5 oder 6, **dadurch gekennzeichnet, dass** der Grundkörper (8) langgestreckt ausgebildet ist und der Sender (2) senkrecht zu der Längserstreckung des Grundkörpers (8) in Richtung auf den Sensor (4) abstrahlend orientiert ist.

8. Vorrichtung nach einem der voranstehenden Ansprüche, **dadurch gekennzeichnet, dass** die Abschattungseinrichtung (6) an einem Ende torsionsfest an einem Körperteil oder Körperbereich festlegbar und an dem anderen Ende frei drehbar ist.

9. Vorrichtung nach einem der voranstehenden Ansprüche, **dadurch gekennzeichnet, dass** der Abschattungseinrichtung (6) zumindest eine Einrichtung (14) zur Festlegung an einem Körperteil oder Körperbereich zugeordnet ist, an der zumindest ein Sensor (4) befestigt ist und an der die Abschattungseinrichtung (6) relativ zu dem Sensor (4) verlagerbar gelagert ist.

10. Vorrichtung nach einem der voranstehenden Ansprüche, **dadurch gekennzeichnet, dass** der Sensor (4) zur Messung der Intensität in einem oder mehreren Frequenzbereichen ausgebildet ist.

11. Vorrichtung nach einem der voranstehenden Ansprüche, **dadurch gekennzeichnet, dass** mehrere Sensoren (4) in Längserstreckung der Abschattungseinrichtung (6) hintereinander angeordnet sind.

12. Vorrichtung nach einem der voranstehenden Ansprüche, **dadurch gekennzeichnet, dass** dem Sensor (4) eine Auswerteeinrichtung (16) zugeordnet ist.

13. Verfahren zur Messung einer Relativverlagerung von Körperteilen oder Körperbereichen zueinander, bei dem eine Abschattungseinrichtung (6) an einer Stelle ortsfest an einem ersten Körperteil oder Körperbereich festgelegt wird, ein Signal von einem Sender (2) in Richtung auf einen Sensor (4) gesendet wird, die an einem als biegsamen Leiter ausgebildeten Grundkörper (8) angeordnete oder ausgebildete Abschattungseinrichtung (6) von dem Signal in Richtung auf den Sensor (4) durchsendet, durchdrungen, durchleuchtet oder angeleuchtet und aufgrund der Relativverlagerung von Körperteilen oder Körperbereichen verlagert wird und ein sich aufgrund der Verlagerung der Abschattungseinrichtung (6) verändernder Einfall auf den Sensor (4) erfasst und in einer Auswerteeinrichtung (16) ausgewertet wird.

14. Verfahren nach Anspruch 13, **dadurch gekennzeichnet, dass** die Abschattungseinrichtung (6) als langgestreckter Grundkörper (8) ausgebildet ist und an einem Ende torsionsfest festgelegt wird und zumindest zwei versetzt zueinander angeordnete Sensoren (4) neben der Abschattungseinrichtung (6) angeordnet sind und die Drehrichtung durch Auswertung der Phasenverschiebung der Sensorsignale bestimmt wird.

15. Verfahren nach Anspruch 13 oder 14, **dadurch gekennzeichnet, dass** mehrere Abschattungseinrichtungen (6) in Verlagerungsrichtung hintereinander angeordnet sind und über den Sensor (4) eine Längenmessung durchgeführt wird.

16. Verfahren nach einem der Ansprüche 13 bis 15, **dadurch gekennzeichnet, dass** das Signal in einen als Leiter ausgebildeten Grundkörper (8) entlang dessen Längserstreckung eingespeist und in Richtung auf den Sensor (4) aus dem Leiter ausgekoppelt wird, wobei an dem Leiter die zumindest eine Abschattungseinrichtung (6) angeordnet oder ausgebildet ist.

## Claims

1. A device for determining relative displacements of body parts or body areas, comprising a transmitter (2) and a sensor (4) assigned to the transmitter (2), whereas between the transmitter (2) and the sensor (4) at least one shadowing apparatus (6) is arranged, which is arranged at, or embodied on, a main body (8) embodied as a conductor, the shadowing apparatus (6) is secured to at least one body part or body area and is displaceable relative to the sensor (4) and/or the transmitter (2), the transmitter (2) is arranged next to the conductor and irradiates the latter or feeds the signal in the longitudinal extent of the conductor into the latter, whereas the main body (8) is flexible and the shadowing apparatus (6) is transmitted through, penetrated, transilluminated or illuminated in the direction of the sensor.

2. The device as claimed in claim 1, **characterized in that** the transmitter (2) is embodied as a light source, magnet, emitter or transmitter emitting electromagnetic waves.

3. The device as claimed in claim 1 or 2, **characterized in that** the shadowing apparatus (6) is arranged between the sensor (4) and the transmitter (2) directed to the sensor (4).

4. The device as claimed in one of the preceding claims, **characterized in that** a plurality of shadowing apparatuses (6) are arranged in succession in the displacement direction.

5. The device as claimed in one of the preceding claims, **characterized in that** the shadowing apparatus (6) is embodied as a coating applied to a main body (8), as a polarization filter or as a region of the main body (8) with deviating transmittance or emission, deviating field line alignment or deviating magnetic flux.

6. The device as claimed in claim 5, **characterized in that** the main body (8) has a substantially round cross section.

7. The device as claimed in claim 5 or 6, **characterized in that** the main body (8) has an elongate embodiment and the transmitter (2) is oriented perpendicular to the longitudinal extent of the main body (8) in a manner emitting in the direction of the sensor (4).

8. The device as claimed in one of the preceding claims, **characterized in that** the shadowing apparatus (6) is securable to a body part or body area in a torsion resistant manner at one end and freely rotatable at the other end.

9. The device as claimed in one of the preceding claims, **characterized in that** at least one apparatus (14) is assigned to the shadowing apparatus (6), on which at least one apparatus, which is to be secured on a body part or body area, at least one sensor (4) is fastened and the shadowing apparatus (6) is mounted in a manner displaceable relative to the sensor (4).

10. The device as claimed in one of the preceding claims, **characterized in that** the sensor (4) is embodied for measuring the intensity in one or more frequency ranges.

11. The device as claimed in one of the preceding claims, **characterized in that** a plurality of sensors (4) are arranged in succession in the longitudinal extent of the shadowing apparatus (6).

12. The device as claimed in one of the preceding claims, **characterized in that** an evaluation apparatus (16) is assigned to the sensor (4).

13. A method for measuring a relative displacement of body parts or body areas in relation to one another, in which a shadowing apparatus (6) is secured in a stationary manner to a first body part or body area at one point, a signal is transmitted in the direction of a sensor (4), the shadowing apparatus (6), which is arranged at or embodied on a main body (8), embodied as a flexible conductor, is transmitted through, penetrated, transilluminated or illuminated by the signal in the direction of the sensor (4) and is displaced due to the relative displacement of body parts or body areas and a changing incidence on the sensor (4) due to the displacement of the shadowing apparatus (6) is registered and evaluated in an evaluation apparatus (16).

14. The method as claimed in claim 13, **characterized in that** the shadowing apparatus (6) is embodied as an elongate main body (8) and secured in a torsion resistant manner at one end and at least two sensors (4) arranged offset in relation to one another are arranged next to the shadowing apparatus (6) and the rotational direction is determined by evaluating the phase shift of the sensor signals.

15. The method as claimed in claim 13 or 14, **characterized in that** a plurality of shadowing apparatuses (6) are arranged in succession in the displacement direction and a length measurement is performed by means of the sensor (4).

16. The method as claimed in one of claims 13 to 15, **characterized in that** the signal is fed into a main body (8), embodied as a conductor, along the longitudinal extent thereof and decoupled from the conductor in the direction of the sensor (4), wherein the at least one shadowing apparatus (6) is arranged at, or embodied on, the conductor.

## Revendications

1. Dispositif de détermination de déplacements relatifs des parties corporelles ou de zones corporelles les unes par rapport aux autres, comportant un émetteur (2) et un capteur (4) associé à l'émetteur (2), dans lequel est prévu, entre l'émetteur (2) et le capteur (4), au moins un organe d'ombrage (6) mobile par rapport au capteur (4) et/ou à l'émetteur (2) et agencé ou réalisé sur un corps de base (8) réalisé sous forme de conducteur, organe qui est susceptible d'être immobilisé sur au moins une partie corporelle ou zone corporelle, et l'émetteur (2) est agencé à côté du conducteur et émet un rayonnement sur celui-ci ou injecte le signal en direction longitudinale du conducteur dans celui-ci, dans lequel
le corps de base (8) est réalisé flexible et l'organe d'ombrage (6) est traversé par le rayonnement, pénétré, traversé par une lumière ou illuminé en direction du capteur (4).

2. Dispositif selon la revendication 1, **caractérisé en ce que** l'émetteur (2) est réalisé sous forme de source lumineuse, d'aimant, d'élément rayonneur ou d'émetteur émettant des ondes électromagnétiques.

3. Dispositif selon la revendication 1 ou 2, **caractérisé en ce que** l'organe d'ombrage (6) est agencé entre le capteur (4) et l'émetteur (2) dirigé vers le capteur (4).

4. Dispositif selon l'une des revendications précédentes, **caractérisé en ce que** plusieurs organes d'ombrage (6) sont agencés les uns derrière les autres en direction de déplacement.

5. Dispositif selon l'une des revendications précédentes, **caractérisé en ce que** l'organe d'ombrage (6) est réalisé sous la forme d'un revêtement déposé sur un corps de base (8), d'un filtre de polarisation ou d'une zone du corps de base (8) présentant une déviation quant à la perméabilité ou au rayonnement, à l'orientation des lignes de champ ou au flux magnétique.

6. Dispositif selon la revendication 5, **caractérisé en ce que** le corps de base (8) présente une section transversale sensiblement ronde.

7. Dispositif selon la revendication 5 ou 6, **caractérisé en ce que** le corps de base (8) est réalisé allongé et l'émetteur (2) est orienté de manière à rayonner perpendiculairement à l'extension longitudinale du corps de base en direction du capteur (4).

8. Dispositif selon l'une des revendications précédentes, **caractérisé en ce que** l'organe d'ombrage (6) peut être immobilisé par une extrémité solidairement en torsion à une partie corporelle ou zone corporelle et peut librement tourner à l'autre extrémité.

9. Dispositif selon l'une des revendications précédentes, **caractérisé en ce qu'**à l'organe d'ombrage (6) est associé au moins un organe (14) d'immobilisation sur une partie corporelle ou une zone corporelle, sur lequel est fixé au moins un capteur (4) et sur lequel l'organe d'ombrage (6) est monté mobile par rapport au capteur (4).

10. Dispositif selon l'une des revendications précédentes, **caractérisé en ce que** le capteur (4) est réalisé pour mesurer l'intensité dans une ou dans plusieurs plages de fréquences.

11. Dispositif selon l'une des revendications précédentes, **caractérisé en ce que** plusieurs capteurs (4) sont agencés les uns derrière les autres selon l'extension longitudinale de l'organe d'ombrage (6).

12. Dispositif selon l'une des revendications précédentes, **caractérisé en ce qu'**un organe d'évaluation (16) est associé au capteur (4).

13. Procédé de mesure d'un déplacement relatif de parties corporelles ou de zones corporelles les unes par rapport aux autres, dans lequel on immobilise un organe d'ombrage (6) à un emplacement de façon stationnaire sur une première partie corporelle ou zone corporelle, on fait émettre un signal par un émetteur (2) en direction d'un capteur (4), l'organe d'ombrage (6) agencé ou réalisé sur un corps de base (8) réalisé sous forme de conducteur flexible est traversé par le signal en direction du capteur (4), ou bien il est, pénétré, traversé par une lumière ou illuminé, et il est déplacé en raison du déplacement relatif de parties corporelles ou de zones corporelles, et une incidence qui se modifie en raison du déplacement de l'organe d'ombrage (6) sur le capteur (4) est détectée et évaluée dans un orange d'évaluation.

14. Procédé selon la revendication 13, **caractérisé en ce que** l'organe d'ombrage (6) est réalisé sous forme de corps de base allongé (8) et est immobilisé par une extrémité solidairement en torsion, et au moins deux capteurs (4) agencés en décalage l'un par rapport à l'autre sont agencés à côté de l'organe d'ombrage (6), et la direction de rotation est déterminée par l'évaluation du déphasage des signaux du capteur.

15. Procédé selon la revendication 13 ou 14, **caractérisé en ce que** plusieurs organes d'ombrage (6) sont agencés les uns derrière les autres en direction de déplacement et une mesure de longueur est réalisée par le capteur (4).

16. Procédé selon l'une des revendications 13 à 15, **caractérisé en ce que** le signal est injecté dans un corps de base (8) réalisé sous forme de conducteur, le long de son extension longitudinale, et il sort hors du conducteur en direction du capteur (4), ledit au moins un organe d'ombrage (6) étant agencé ou réalisé sur le conducteur.
